# FASCICULE DE BREVET EUROPEEN

(11) **EP 1 723 958 B1**
(45) Date de publication et mention de la délivrance du brevet: **26.06.2013**
(21) Numéro de dépôt: 06290795.1
(22) Date de dépôt: 16.05.2006
(51) Int. Cl.: A61K 31/713, A61K 8/60

(54) **Vectorisation d'ARN double brin par des particules cationiques et leur utilisation topique**
Doppelsträngige-RNA Vektorisierung mittels kationischen Partikeln und ihre topische Anwendung
Double strand RNA vectorisation with cationic particles and their topical use

(30) Priorité: 19.05.2005 FR 0551301
(43) Date de publication de la demande: 22.11.2006
(73) Titulaire: L'Oréal, 75008 Paris (FR)
(72) Inventeur: Collin-Djangone, Christine, 60110 Amblainville (FR); Simonnet, Jean-Thierry, 94240 Cachan (FR)
(74) Mandataire: Bernstein, Claire Jacqueline

(56) Documents cités:
- WO-A-03/101376
- WO-A-03/106636
- WO-A-2004/046354
- STEIN C A ET AL: "A critical assessment of the potential of short interfering RNA therapeutics" DRUG DISCOVERY TODAY: TECHNOLOGIES, ELSEVIER, vol. 2, no. 1, avril 2005 (2005-04), pages 27-31, XP004985725 ISSN: 1740-6749

## Description

La présente invention se rapporte à la vectorisation de dsRNA par des particules cationiques choisies parmi des micelles de tensioactifs et des micelles de polymères blocs ainsi qu'à des compositions topiques pour la peau, les muqueuses ou les yeux comprenant l'association d'au moins un dsRNA et d'au moins une particule cationique.

L'utilisation de substances présentant une activité spécifique, telle qu'une activité biologique spécifique, dans le domaine cosmétique tel que le soin de la peau ou le soin capillaire, mais aussi dans les domaines dermatologique et pharmaceutique, est recherchée.
Depuis peu, l'utilisation de dsRNA et, plus particulièrement, de siRNA (de 12 à 40 nucléotides) permet d'obtenir une activité d'inhibition spécifique de la synthèse d'une protéine cible.
Le mécanisme moléculaire mis en jeu fait intervenir des fragments d'ARN double brin constitués de 12 à 40 nucléotides. La dégradation de l'ARNm cible est obtenue par l'activation du complexe RISC (RNA Induced Silencing Complex) effective par la fixation du brin anti-sens du dsRNA sur l'ARNm. Ces oligonucléotides d'ARN double brin, sont aussi appelés dsRNA ou encore siRNA (pour «short interfering» RNA), voir Tuschl T. Chem. Biochem. 2001 ;2 :239-245, Nykanen A & al Cell 2001 107 309-321, Dorsett Y. Nature, April 2004, Vol.3, page 318-329 et Downward J. BMJ 2004, 328, 1245-1248.

Cependant, le problème de la pénétration lors de l'application topique de ces siRNA, dont le poids moléculaire peut être d'environ 15-17 kD, se pose et demande à être optimisé. En effet, pour que l'activité spécifique relative au siRNA sélectionnée soit effective, il est nécessaire que celui-ci pénètre dans la cellule cible (par exemple les kératinocytes ou les mélanocytes...) jusque dans son cytoplasme. Or le *stratum corneum,* siège de la fonction barrière cutanée, est difficile à pénétrer. Il reste donc à rechercher une solution permettant la pénétration de siRNA dans la peau.

De manière inattendue, la demanderesse a constaté que l'association de siRNA avec des particules cationiques permet d'améliorer très significativement leur pénétration dans les cellules cibles d'un tissu tel que la peau. Une fois pénétré, le siRNA devient actif par sa fixation sur le RISC. La pénétration peut être évaluée à l'aide de marqueur fluorescent fixé sur le siRNA et son activité par la quantification du messager ciblé par PCR quantitative ou par le dosage de la protéine correspondante au messager ciblé. Les particules cationiques de l'invention, peuvent être des micelles de tensioactifs, ou des micelles de polymères blocs. D'une façon générale, la taille moyenne des particules de l'invention, doit être inférieure au micromètre.

La demande WO 03/101376 décrit des compositions cosmétiques comprenant au moins un oligonucléotide d'ARN double brin. Dans cette demande, l'oligonucléotide est nécessairement complexé avec un polymère cationique comme par exemple la PEI ou le chitosan, ce complexe oligonucléotide-polymère cationique pouvant ensuite être encapsulé dans des liposomes ou des niosomes et/ou adsorbé à la surface de particules telles que les liposomes, niosomes, oléosomes, nanosphères et nanocapsules.
Ainsi, dans ce document, le dsRNA est nécessairement complexé avec un polymère cationique avant d'être associé à un type de particule.
De plus, les micelles de tensioactifs ne sont pas décrites comme solution galénique performante pour la vectorisation de siRNA.

Dans l'article: Intercellular adhesion molecule-1 suppression in skin by topical delivery of anti-sense oligonucleotides de Mehta et al (J.Invest.Dermatol 115:805-812, 2000*),* les auteurs véhiculent de l'ADN simple brin dans une émulsion contenant 25% de tensioactifs (10% de glycéryl stearate et 15% de stéarate 40 OE). Outre le fait que cet article ne décrive pas les siRNA, la solution galénique proposée n'est pas compatible avec une bonne tolérance cutanée. En effet, les 25% de tensioactifs auront pour effet de détruire la fonction barrière de la peau, augmentant ainsi sa perméabilité aux éléments extérieurs et favorisant dramatiquement sa déshydratation : résultats qui ne sont pas recherchés dans les domaines d'application envisagés par la présente invention.

Dans la publication : lipidic carriers of siRNA : differences in the formulation, cell uptake and delivery with plasmid DNA de Spagnou et al (Biochemistry, 43:13348-56, 2004*)**,* les auteurs utilisent des liposomes cationiques tels que la lipofectamine 2000 ou des liposomes de DOPE « cationisés » avec du cholesteryloxycarbonyl3-7 diazanonane 1-9 diamine (CDAN). Pour des raisons d'innocuité, la lipofectamine ne peut être utilisée sur peau humaine et on cherchera des solutions plus faciles à mettre en oeuvre et économiquement compatibles avec la mise sur le marché du produit.

D'autres publications (Yano Junichi et al. Clinical Cancer Research, 2004, Spagnou et al. Biochemistry, 2004**,** et Ma Zheng et al. Biochemical and Biophysical Research Communication, 2004) ont proposé d'associer des siRAN à des vésicules cationiques constituées de lipides à chaînes oleyles comportant des insaturations les rendant très sensibles à l'oxydation et de phospholipides, ces vésicules sont destinées à être utilisées immédiatement après préparation car ne sont pas stables dans le temps.

La demande de brevet WO 2004/046354 décrit l'utilisation de siRNA pour les dysfonctionnements cutanés et de préférence au niveau du derme. Les compositions décrites telles que les PIT, les émulsions W/O, O/W et W/O/W sont toutes non ioniques. Il n'est pas fait état dans cette demande de brevet de l'intérêt majeur et essentiel d'utiliser des tensioactifs cationiques dans des particules cationiques pour favoriser la pénétration des siRNA dans les structures cutanées. Par ailleurs, si les PIT peuvent être inférieures au µm, elles sont nécessairement non ioniques, comme décrit dans le brevet WO96/28132.

Le document WO 03/106636 décrit l'association d'un polynucléotide avec un tensioactif cationique tel que le Cétyl Tri Ammonium Chloride pour former un complexe. Il est nécessaire ensuite de stabiliser ce complexe par une étape d'incubation à une température comprise entre 35 et 50°C.
Dans un second temps, il est possible de déshydrater le mélange pour en obtenir une poudre qui sera ensuite diluée dans un solvant choisi, avant usage (injection). Il est également possible, une fois le complexe formé, de le stabiliser par l'ajout d'un lipide amphiphile qui peut être un tensioactif non ionique. Cette association ternaire est ensuite déshydratée pour obtenir une poudre qui sera ensuite diluée dans une solution aqueuse adaptée.
La demande WO 03/106636 ne précise pas qu'il faille se situer au dessus de la CMC (Concentration Micellaire Critique) du tensioactif cationique pour former le complexe polynucléotide/tensioactif cationique, le complexe pouvant se former en dessous de la CMC.
Ce n'est qu'une fois ce complexe formé qu'il est envisagé l'ajout d'un autre composé amphiphile qui pourra former des micelles. On a ainsi l'intégration d'un complexe Polynucléotideltensioactif cationique dans des micelles d'amphiphiles.
Ainsi dans ce document, il n'est pas question d'associer des particules cationiques à un polynucléotide.

Ainsi, la présente demande décrit en premier lieu à une composition topique ou oculaire comprenant au moins un oligonucléotide d'ARN double brin, caractérisée en ce que l'oligonucléotide d'ARN double brin est associé à au moins une particule cationique d'une taille inférieure ou égale à 1 µm, de potentiel zeta compris entre 10 et 80 mV choisie parmi les micelles de tensioactifs, en particulier, les micelles de tensioactifs amphiphiles non ioniques et de tensioactifs cationiques, les micelles de polymères blocs, en particulier, les micelles de polymère bloc amphiphile cationique, les micelles de polymère bloc amphiphile non ionique et de polymère bloc amphiphile cationique et les micelles de polymère bloc amphiphile non ionique et de tensioactif cationique.

Par composition topique, on entend une composition adaptée à l'application sur la surface du corps, c'est-à-dire, la peau, les phanères, les muqueuses et des yeux.

La présente invention concerne l'association de particules cationiques avec au moins un dsRNA qui va adhérer à la surface de la particule, celle-ci servant de véhicule pour le faire pénétrer dans les structures cutanées et dans les cellules cibles de la peau, des muqueuses ou encore des yeux pour des applications cosmétiques, dermatologiques et/ou ophtalmiques.

Les particules cationiques selon l'invention sont des particules ayant une taille inférieure ou égale à 1 µm, de préférence inférieure ou égale à 500 nm, encore préférentiellement inférieure ou égale à 300 nm, mesurable avec, par exemple, un granulométre laser type B190 plus de la société Brookhaven, et ayant un potentiel zeta compris entre 10 et 80 mV pouvant être mesuré avec un zetamètre type DELSA 440 de la société Coultronics.

Sont listées ci-après une liste non exhaustive des particules cationiques utilisables selon l'invention.

### Micelles de tensioactifs

Pour rappel, les micelles sont les agrégats que forment spontanément les molécules amphiphiles lorsqu'elles sont solubilisées dans l'eau ou l'huile, au-delà d'une certaine concentration dite critique : la CMC.
Les micelles utilisables dans le cadre de l'invention sont constituées d'au moins un tensioactif cationique. Ce tensioactif cationique peut être associé à un ou plusieurs tensioactifs amphiphiles non ioniques.
L'homme du métier sélectionnera avantageusement les tensioactifs non ioniques et cationiques dans le McCutcheons 1998 « emulsifiers and detergents » ainsi que dans les éditions suivantes.
A titre d'exemples non limitatifs, les tensioactifs cationiques utilisables dans le cadre de l'invention sont listés ci-après.

Sans que ce soit limitatif, les tensioactifs non ioniques utilisables sont : les alkyls et polyalkyls (C6 à C30, saturé ou non, ramifié ou non) ester ou ether de POE, de glycérol et de polyglycérol, de sorbitan oxyéthyléné ou non, de sucrose, de glucose oxyethyléné ou non, de maltose, de POP-POE.

En cas de mélange entre tensioactifs non ioniques et tensioactifs cationiques, leurs proportions respectives, en poids, seront comprises entre 99/1 et 1/99.

Le taux de tensioactifs formant les micelles sera dépendant de la CMC de ceux-ci. Cependant, dans le cadre de l'invention, la concentration en tensioactifs micellaires sera comprise entre 0.1 et 10% et de préférence entre 0.2 et 5% en poids par rapport au poids total de la composition.

### Micelles de polymère bloc

Les micelles de polymères blocs amphiphiles peuvent être préparées selon le procédé décrit dans le document WO 04/035013.

Les copolymères blocs utiles pour la préparation des micelles associés aux dsRNA selon l'invention sont notamment des polymères blocs amphiphiles, préférentiellement non ioniques, diblocs ou triblocs, qui peuvent former au contact de l'eau des micelles. Ils sont notamment de type dibloc (A-B) ou tribloc (A-B-A), A correspondant à un bloc polymérique hydrophile non ionique et B à un bloc polymérique hydrophobe. Le poids moléculaire des polymères peut être compris entre 1000 et 100.000 et le rapport A/B peut être compris entre 1/100 et 50/1.

Le bloc polymérique hydrophile non ionique peut être choisi parmi le polyoxyde d'éthylène (POE) et la polyvinylpyrrolidone (PVP), le poly acide acrylique (PAA).

Le bloc polymérique hydrophobe peut être choisi parmi le polystyrène, le poly(tert.-butylstyrène), le poly(méthylméthacrylate), le poly(éthylacrylate), le poly(butylacrylate), le poly(butylméthacrylate), le poly(vinylacétate), les polycaprolactones, les polycaprolactames, les polydiméthylsiloxanes, les polyoxydes d'alkylène en C3-C6, le poly(acide aspartique), le poly(acide lactique), le poly(acide glycolique), la polyleucine, les polybutadiènes, les polyéthylènes, les polypropylènes, les polybutylènes.

Le copolymère bloc est choisi de préférence parmi les copolymères blocs suivants :
- polyoxyde de propylène/polyoxyde d'ethylène
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

Dans le cadre de l'invention, il est nécessaire d'ajouter à la composition micellaire :
- un polymère bloc amphiphile cationique dont l'un des blocs est cationique et peut être choisi parmi, à titre d'exemple :
   - Poly methacrylate de trimethyl ethyl ammonium ;
   - Polymethacrylate de dimethyl amino ethyl quaternisé ;
   - Poly methyl vinyl imidazolium ;
   - Poly vinyl benzyl trimethylamonium chlorure.
   l'association d'un polymère bloc amphiphile non ionique avec un polymère blocs amphiphile cationique est telle que le rapport entre les deux sera compris entre 99/1 et 1/99 ;
   et/ou
- au moins un tensioactif cationique tel que listé ci-après.
   Dans ce cas, le rapport respectif entre le polymère bloc amphiphile non ionique et le tensioactif cationique sera compris entre 50/50 et 99/1.

Dans le cadre de l'invention, la concentration en polymères blocs micellaires associés ou non à un tensioactif cationique, sera comprise entre 0,1 et 10% et, de préférence, entre 0,2 et 5% en poids par rapport au poids total de la composition.

Dans une variante de l'invention, on peut également former des micelles constituées de polymères blocs amphiphile cationique tels que décrits ci-dessus.

>

>

>

### Tensioactifs cationiques utilisables pour la préparation des particules cationiques de l'invention

Les tensioactifs cationiques qui peuvent être utilisés selon l'invention sont listés ci-après, cette liste est non limitative.

Les lipides amphiphiles cationiques sont choisis, de préférence, dans le groupe formé par les sels d'ammonium quaternaire.

Les sels d'ammonium quaternaires sont par exemple :
- ceux qui présentent la formule générale (IV) suivante : 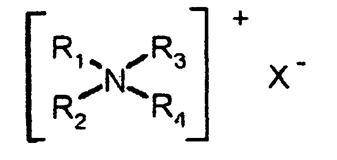 dans laquelle les radicaux R1 à R4, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C2-C6), alkylamide, alkyl(C12-C22)amido alkyle(C2-C6), alkyl(C12-C22)acétate, hydroxyalkyle, comportant environ de 1 à 30 atomes de carbone; X est un anion choisi dans le groupe des halogénures, phosphates, acétates, lactates, alkyl(C2-C6)sulfates, alkyl-ou-alkylarylsulfonates,
- les sels d'ammonium quaternaire de l'imidazolinium, comme par exemple celui de formule (V) suivante : 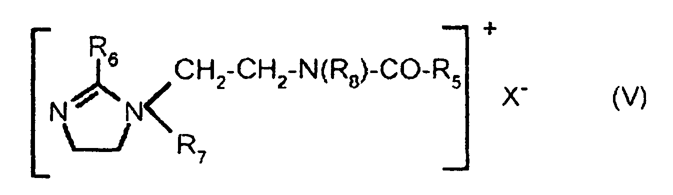 dans laquelle R5 représente un radical alcényle ou alkyle comportant de 8 à 30 atomes de carbone par exemple dérivés des acides gras du suif, R6 représente un atome d'hydrogène, un radical alkyle en C1-C4 ou un radical alcényle ou alkyle comportant de 8 à 30 atomes de carbone, R7 représente un radical alkyle en C1- C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates. De préférence, R5 et R6 désignent un mélange de radicaux alcényle ou alkyle comportant de 12 à 21 atomes de carbone par exemple dérivés des acides gras du suif, R7 désigne méthyle, R8 désigne hydrogène. Un tel produit est par exemple commercialisé sous la dénomination «REWOQUAT W 75» par la société REWO,
- les sels de diammonium quaternaire de formule (VI) : 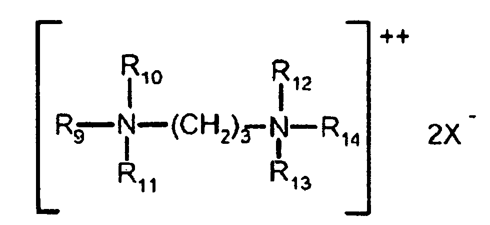 dans laquelle R9 désigne un radical aliphatique comportant environ de 16 à 30 atomes de carbone, R10, R11, R12, R13 et R14 **,** identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester
   Les sels d'ammonium quaternaire contenant au moins une fonction ester utilisables selon l'invention sont par exemple ceux de formule (VII) suivante : 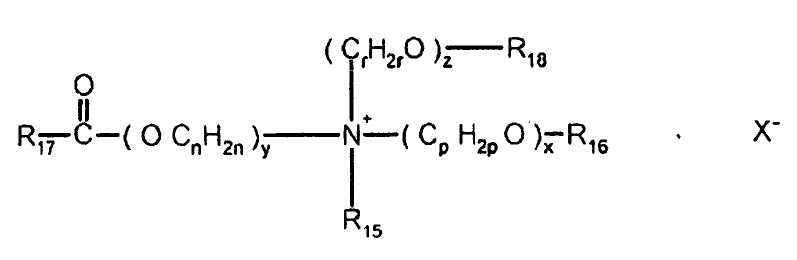 dans laquelle :
- R15 est choisi parmi les radicaux alkyles en C1-C6 et les radicaux hydroxyalkyles ou dihydroxyalkyles en C1-C6 ;
- R16 est choisi parmi :
- le radical 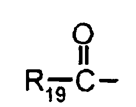
- les radicaux R20 hydrocarbonés en C1-C22 linéaires ou ramifiés, saturés ou insaturés,
- l'atome d'hydrogène,
- R18 est choisi parmi :
- le radical 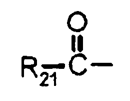
- les radicaux R22 hydrocarbonés en C1-C6 linéaires ou ramifiés, saturés ou insaturés,
- l'atome d'hydrogène,
- R17, R19 et R21, identiques ou différents, sont choisis parmi les radicaux hydrocarbonés en C7-C21, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ; sous réserve que la somme x + y + z vaut de 1 à 15, que lorsque x vaut 0 alors R16 désigne R20 et que lorsque z vaut 0 alors R18 désigne R22.
Les radicaux alkyles R15 peuvent être linéaires ou ramifiés et plus particulièrement linéaires. De préférence R15 désigne un radical méthyle, éthyle, hydroxyéthyle ou dihydroxypropyle et plus particulièrement un radical méthyle ou éthyle. Avantageusement, la somme x + y + z vaut de 1 à 10.
Lorsque R16 est un radical R20 hydrocarboné, il peut être long et avoir de 12 à 22 atomes de carbone ou court et avoir de 1 à 3 atomes de carbone.
Lorsque R18 est un radical R22 hydrocarboné, il a de préférence 1 à 3 atomes de carbone.
Avantageusement, R17, R19 et R21, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.
De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X- est encore plus particulièrement le chlorure ou le méthylsulfate.

On utilise plus particulièrement les sels d'ammonium de formule (VII) dans laquelle :
- R15 désigne un radical méthyle ou éthyle,
- x et y sont égaux à 1 ;
- z est égal à 0 ou 1 ;
- n, p et r sont égaux à 2 ;
- R16 est choisi parmi :
- le radical 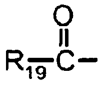
- les radicaux méthyle, éthyle ou hydrocarbonés en C14-C22
- l'atome d'hydrogène ;
- R18 est choisi parmi :
- le radical 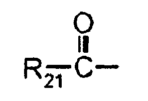
- l'atome d'hydrogène ;
R17, R19 et R21, identiques ou différents, sont choisis parmi les radicaux hydrocarbonés en C13-C17, linéaires ou ramifiés, saturés ou insaturés et de préférence parmi les radicaux alkyles et alcényle en C13-C17, linéaires ou ramifiés, saturés ou insaturés.
Avantageusement, les radicaux hydrocarbonés sont linéaires.

On peut citer par exemple les composés de formule (VII) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

Lorsque le composé contient plusieurs radicaux acyles, ces derniers peuvent être identiques ou différents.
Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.
De tels composés sont par exemple commercialisés sous les dénominations DEHYQUART par la société HENKEL, STEPANQUAT par la société STEPAN, NOXAMIUM par la société CECA, REWOQUAT WE 18 par la société REWOWITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60 % de méthylsulfate de diacyloxyéthyl hydroxyéthyl méthyl ammonium et 15 à 30 % de méthylsulfate de triacyloxyéthyl méthyl ammonium, les radicaux acyles ayant de 14 à 18 atomes de carbone et provenant d'huile de palme éventuellement partiellement hydrogénée. On peut aussi utiliser les sels d'ammonium contenant au moins une fonction ester décrits dans les brevets US-A-4874554 et US-A-4137180.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Selon l'invention, le chlorure ou le bromure de béhényltriméthylammonium et le CTAB (cétyltrimethylammonium bromine) sont les sels d'ammonium quaternaires les plus particulièrement préférés.

Les particules de la présente invention peuvent être introduites dans tous supports galéniques à visée cosmétique, dermatologique et ophtalmique. A titre d'exemple, on citera les lotions, les serums, les gels et tous les type d'émulsions.

Les oligonucléotides d'ARN double brin (ou encore dsRNA pour double stranded RNA, siRNA pour short interfering RNA) utilisables selon la présente invention sont des fragments d'acides nucléiques doubles brins capables d'inhiber totalement ou partiellement l'expression d'un gène dans une cellule eucaryote, ces oligonucléotides d'ARN double brin comportent généralement entre 12 et 40 nucléotides, préférentiellement de 20 à 25 nucléotides. Ces oligonucléotides double brin sont composés d'un brin sens et d'un brin anti-sens correspondant à la séquence de l'ARN messager cible à dégrader. L'oligonucléotide d'ARN double brin présente des extrémités franches ou non appariées de 2 à 6 nucléotides.
Les oligonucléotides d'ARN double brin selon l'invention pourront être des oligonucléotides d'ARN double brin comportant un ou plusieurs nucléotides modifiés par substitution, délétion ou insertion, ces modifications seront telles que la séquence l'oligonucléotide d'ARN double brin lui permettra de reconnaître spécifiquement un fragment de l'ARNm cible du mécanisme de dégradation.

Les oligonucléotides d'ARN double brin pourront également présenter un squelette modifié lui conférant, par exemple, une meilleure stabilité.
Par exemple, les liaisons phosphodiester des brins d'ARN naturels peuvent être modifiées pour inclure au moins un atome d'azote ou de soufre. De plus, les oligonucléotides d'ARN double brin selon l'invention peuvent comprendre des bases autres que les 4 bases classiques.
La structure en double brin de l'oligonucléotide d'ARN double brin peut être obtenue par l'appariement de deux brins simples d'ARN complémentaires ou bien par un unique brin simple d'ARN "autocomplémentaire", c'est-à-dire comprenant deux fragments de séquences complémentaires pouvant s'apparier par repliement du brin simple pour former une double hélice.
A titre d'exemple d'oligonucléotides d'ARN double brin, on peut citer ceux décrits dans les demandes de brevet WO 00/44895, WO 01/36646, WO 99/32619, WO 01/29058, WO 00/44914 ou encore WO 03/101376.
Il peut également s'agir d'oligonucléotides d'ARN double brin dit « stealth RNA » tels que ceux vendus par la société Invitrogen et notamment ceux décrits dans les demandes de brevets US2004054155 et US2004014956 qui sont des oligonucléotides d'ARN double brin modifiés de telle sorte qu'une de ses extrémités, par exemple et de façon non limitative, comporte un groupement 2'-O-méthyl.

Les oligonucléotides d'ARN double brin peuvent être synthétisés selon de nombreuses méthodes de synthèse *in vivo* ou *in vitro* de façon manuelle ou automatique.

Les méthodes de synthèse *in vitro* peuvent être chimiques ou enzymatiques, par exemple en utilisant une ARN polymérase (à titre d'exemple de T3, T7 ou SP6) qui réalisera la transcription d'un modèle de séquence d'ADN (ou d'ADNc) choisi.

De nombreuses méthodes de synthèse *in vivo* d'ARN double brin sont décrites dans la littérature, elles peuvent être réalisées dans divers types cellulaires de bactéries ou d'organismes supérieurs (Sambrook et al. Molecular Cloning, A Laboratory Manual, Second Edition (1989), DNA cloning, volume I and II, D. N. Glover (ed. 1985), Oliginucleotide Synthesis, M. J. Gaits (ed. 1984), Nucleic Acid Hybridation, B. D. Hames and S. J. Higgins (ed.1984), Transcription and Translation B. D. Hames and S. J. Higgins (ed.1984), Animal Cell Culture, R. I. Freshney (ed. 1986), Immobilised Cells and Enzymes, IRL Press (1986), B. Pertal, A Practical Guide to Molecular Cloning (1984), Gene Transfer Vectors for Mammalian Cells, J. H. Miller and M. P. Calos, Cold Spring Harbor Laboratory (ed. 1987), Methods in Enzymology, vo1.154, Wu and Grossman, and 155, Wu, Mayer and Walker (1987), Immunochemical Methods in Cell and Molecular Biology, Academic Press, London, Scopes (1987), Protein Purification: Principle and Practice, 2nd ed., Springer-Verlag, N.-Y. and Handbook of Experimental Immunology, vol.I-IV, C. D. Weir and C. C. Blackwell (1986)). On pourra également se référer aux méthodes de synthèses décrites dans les demandes de brevet WO01/36646, WO01/75164 et US20030088087.

L'homme du métier choisira une concentration en oligonucléotide d'ARN double brin adapté à l'usage visé et à l'activité de l'oligonucléotide choisi. Sans que ce soit limitatif, la concentration en oligonucléotide d'ARN double brin pourra être comprise entre 1 nM et 100 mM.

Un second objet de la présente demande se rapporte à l'utilisation topique ou oculaire d'une composition comprenant au moins un oligonucléotide d'ARN double brin et d'au moins une particule cationique d'une taille inférieure ou égale à 1 µm, de préférence, inférieure ou égale à 500 nm ou 300 nm, de potentiel zeta compris entre 10 et 80 mV choisie parmi les micelles de tensioactifs, les micelles de polymères blocs.

Un autre objet de la présente demande se rapporte à un procédé de traitement cosmétique comprenant l'application topique ou oculaire d'une composition comprenant au moins un oligonucléotide d'ARN double brin et d'au moins une particule cationique d'une taille inférieure ou égale à 1 µm, de potentiel zeta compris entre 10 et 80 mV choisie parmi les micelles de tensioactifs, les micelles de polymères blocs.

A des fins de soin cosmétique, dermatologique, ophtalmique ou pharmaceutique, on cherche à éviter les méthodes invasives telles que les injections sous cutanées ou oculaires. Les particules cationiques associées au siRNA selon la présente invention peuvent être appliquées topiquement sur la peau, les muqueuses ou dans l'oeil, en suspension ou incorporées dans un support cosmocentrique acceptable, comme des lotions, sérum, gels émulsionnés ou non, des émulsions huile/eau, eau/huile, multiples, les microémulsions... On veillera à ce que cette introduction dans un support cosmétique ne nuise pas à la stabilité des particules associées au siRNA.

A titre d'exemple non limitatif, la composition selon l'invention comprenant des dsRNA vectorisés à l'aide de particules cationiques pourra être destinée à prévenir et/ou traiter le vieillissement actinique ou chronologique, les rides, la perte de fermeté cutanée de la peau du visage et/ou du corps, les troubles de la séborrhée, les peaux grasses, les peaux sèches, les taches pigmentaires. Ces compositions peuvent également être destinées à freiner la chute des cheveux, à stimuler leur pousse, à améliorer leur qualité, à limiter ou empêcher la repousse des poils, etc...
Ces compositions pouvant également être destinées à traiter des désordres cutanés ou ophtalmologiques.

Pour favoriser la pénétration des particules associées au siRNA, on pourra modifier la perméabilité de la peau, ainsi, il est possible de contrôler la perméabilité de la peau par la mesure de la PIE (perte insensible en eau) avec un Tewametre TM210 ou un Dermalab - Cortex technology.
On pourra utiliser, à titre d'exemple, les méthodes suivantes :
- stripping (cornéodisque, « vernis »), peeling chimique ou dermabrasion mécanique ;
- pré-traitement par un mélange d'un ou plusieurs solvants ayant un effet délipidant ;
- nettoyage de la peau par un produit moussant détergent ;
- pré et/ou post traitement occlusif soit par exemple en recouvrant la surface de la peau à traiter d'une membrane synthétique étanche (Blenderm par exemple) soit par l'application d'une couche de vaseline. Cela a pour effet de bloquer la PIE naturelle de la peau et de provoquer une sur-hydratation des lipides épidermiques qui deviennent ainsi plus perméable.

Après l'application de la composition de la présente invention, on pourra également utiliser des méthodes connues de l'homme du métier favorisation la pénétration de molécules au sein de la peau telles que, par exemple, la iontophorèse ou l'électroporation.

Enfin, la présente demande décrit l'utilisation de particules cationique d'une taille inférieure ou égale à 1 µm, de potentiel zeta compris entre 10 et 80 mV choisie parmi les micelles de tensioactifs, les micelles de polymères blocs comme agent favorisant la pénétration topique ou oculaire d'au moins un oligonucléotide d'ARN double brin.

Les figures suivantes illustrent les essais décrits dans l'exemple 2:
**1. Ciblage cellulaire dans Episkin-J6**
   **Fig. I-1** **:** Block-It Fluorescent Oligo (DAPI)
   **Fig. I-2** **:** Block-It Fluorescent Oligo + Lipofectamine 2000 (DAPI)
   **Fig. I-3** **:** Block-It Fluorescent Oligo + E2 (x 40, DAPI)
   **Fig. I-4** **:** E2 (x 40, DAPI)
   **Fig. I-5** **:** Block-It Fluorescent Oligo + E2 (x 100, DAPI)
   **Fig. I-6** **:** Block-It Fluorescent Oligo + E2 (x 100)
**II. Ciblage cellulaire dans Episkin-J13**
   **Fig. II-1 :** Block-It Fluorescent Oligo (x 40, DAPI)
   **Figures IIIA et IIIB :** visualisation de l'effet de formulation comprenant de l'octyle glucoside et du CTAB à différentes concentrations sur la viabilité de cellules HaCaT.

### EXEMPLE 1 - FORMULATIONS

### Micelles cationiques:

Exemple :
- Octyl β Glucoside / cetyl triammonium bromide au ratio molaire de 5/1
   E1: 100 mM dans l'eau distillée
   E2: 50 mM dans l'eau distillée
   E3 : 25 mM dans l'eau distillée
   E4: 12.5 mM dans l'eau distillée

Les 2 tensio actifs (non ionique + cationique) sont solubilisés dans l'eau distillée. Une suspension de siRNA (20 µM) est ajoutée entre 1:1 et 1:9 volume (siRNA: micelles) réduisant ainsi la concentration micellaire en proportion.
- Micelles de décyl b glucoside / behenyl triammonium chloride au ratio molaire de 5/1 dans l'eau distillée. Les mêmes concentrations que dans l'exemple précédent sont réalisées.

### EXEMPLE 2 - CIBLAGE CELLULAIRE

Le ciblage cellulaire et tissulaire a été effectué dans le modèle d'épiderme reconstruit développé par EPISKIN SNC. Deux temps de cinétique de croissance du modèle ont été choisis pour l'application topique du complexe micelle/siRNA :
√ 1 application à J6 de croissance de l'épiderme correspondant à un épiderme en début de stratification et de kératinisation.
√ 1 application à J13 de croissance de l'épiderme correspondant à un épiderme stratifié
et kératinisé.
Le siRNA choisi est le Block-It Fluorescent Oligo (20 µM), tel qu'il est décrit dans le manuel « Block-It Transfection Kit » (Réf cat N°: 13750-070, Invitrogen). Il s'agit d'un double brin d'ARN de 25 nucléotides couplé à la fluorescéine. La séquence codée n'a aucune homologie avec le génome humain et le rend donc non fonctionnel. Cet oligonucléotide a été spécifiquement élaboré pour l'étude du ciblage cellulaire. Lorsqu'il est transfecté, il est présent dans le cytoplasme et pénètre aussi dans le noyau cellulaire.

### Préparation du complexe micelle/siRNA :

3 µl de Block-It Fluorescent Oligo (20 µM), tel qu'il est décrit dans le manuel « Block-It Transfection Kit » (Réf cat N°: 13750-070, Invitrogen), sont mélangés à 9 µl de micelles cationiques E2 (Octyl β Glucoside / cetyl triammonium bromide au ratio molaire de 5/1, 50 mM dans l'eau distillée).

### Préparation du mélange Lipofectamine 2000/siRNA :

3 µl de Block-It Fluorescent Oligo (20 µM) sont dilués dans 50 µl d'OptiMEM (Invitrogen) et mélangés à 3 µl de Lipofectamine 2000 (Invitrogen) diluée dans 12 µl d'OptiMEM selon le protocole du fournisseur. La solution est incubée à température ambiante pendant 20 min pour permettre la complexation des liposomes et du siRNA.

### Préparation des solutions de contrôle :

√ 3 µl de Block-It Fluorescent Oligo (20 µM) sont dilués dans 9 µl d'OptiMEM.
√ 9 µl de micelles cationiques E2 (Octyl β Glucoside / cetyl triammonium bromide au ratio molaire de 5/1, 50 mM dans l'eau distillée) sont diluées dans 3 µl d'OptiMEM.

Les différents mélanges et solutions sont alors déposés sur différents échantillons d'épiderme EPISKIN et incubés en émersion 48 h à 37°C/5% CO2 dans du milieu de différenciation fourni avec le kit EPISKIN. Les échantillons sont alors congelés dans un mélange carboglace/Ethanol et coupés à l'aide d'un cryostat (MICROM HM560) en sections de 5 µm. Les coupes sont montés dans du milieu de montage Vectrashield (Vector Laboratories) contenant 1,5 µg de DAPI et visualisés en immunofluorescence avec les filtres appropriés pour la fluorescéine et le DAPI.

### Observations :

√ Epiderme transfectés à J6 : on observe que, alors que la solution de Block-It Fluorescent Oligo **(Figure I-1)** ou le mélange Lipofectamine 2000 **(Figure I-2)** ne permet pas la pénétration de Block-It Fluorescent Oligo au-delà du stratum corneum en formation, le mélange E2/Block-It Fluorescent Oligo permet la pénétration du duplex RNA dans l'épiderme **(Figures I-3, I-5, I-6).** Aucun marquage non spécifique n'est détecté lorque E2 est appliqué seul **(Figures I-4).** Le caractère surprenant de cette observation est souligné par la présence homogène du Block-It Fluorescent Oligo dans toutes les cellules de l'épiderme alors qu'il est connu par l'homme de l'art que la transfection classique n'est efficace que dans les cellules en prolifération. Dans l'épiderme reconstruit, seules les cellules basales prolifèrent tandis que les cellules supra-basales se différencient.
√ Epiderme transfectés à J13 : comme à J6, la solution de Block-It Fluorescent Oligo seule ne pénètre pas le stratum corneum **(Figure II-1).** Le mélange E2/ Block-It Fluorescent Oligo permet la pénétration du duplex fluorescent dans les différentes couches du *stratum corneum* et dans les différentes cellules de l'épiderme **(Figures II-2, II-3)** avec toutefois une efficacité et une homogénéité moindre qu'à J6.

### EXEMPLE 3 - EFFET D'UNE FORMULATION D'OCTYLE GLUCOSIDE ET DE CTAB A DIFFERENTES CONCENTRATIONS SUR LA MORTALITE DE CELLULES HaCaT

L'effet de la formule E2 : *50 mM micelles octyl glucoside* / *CTAB au ratio molaire 5*/*1* est réalisée par incubation de 4 jours selon le protocole suivant sur cellules HaCaT (500 µl de milieu ensemencé à 40 000 cellules/puits, B-It : Block-It Fluorescent Oligo (Invitrogen).

40 000 cellules HaCaT/ puits sont ensemencées en duplicate dans une plaque 24 puits dans 500 µl de DMEM complet + 10% sérum de veau foetal.
1 µl de Block-It Fluorescent Oligo (Invitrogen) est dilué dans 1-15 µl d'une solution micellaire E2 composée de 50mM de micelles octyl glucoside / CTAB au ratio molaire de 5/1 et ajouté dans le milieu de culture soit une concentration finale de solution micellaire 0,1-1,5 mM.
Les cellules sont alors incubées 4 jours à 37°C avec un changement de milieu pour un échantillon des duplicates à J1 puis photographiées.

Les résultats obtenus sont représentés aux **figures IIIA et IIIB.**
Aucune différence n'est observée pour un même traitement entre les cellules avec ou sans changement de milieu à J1.
On observe une forte mortalité pour les concentrations de solution micellaire inférieure à la CMC (env. 1 mM) mettant en évidence l'effet toxique du véhicule sous sa forme libre tandis qu'une bonne viabilité cellulaire est observée au dessus de la CMC (1.5 mM).

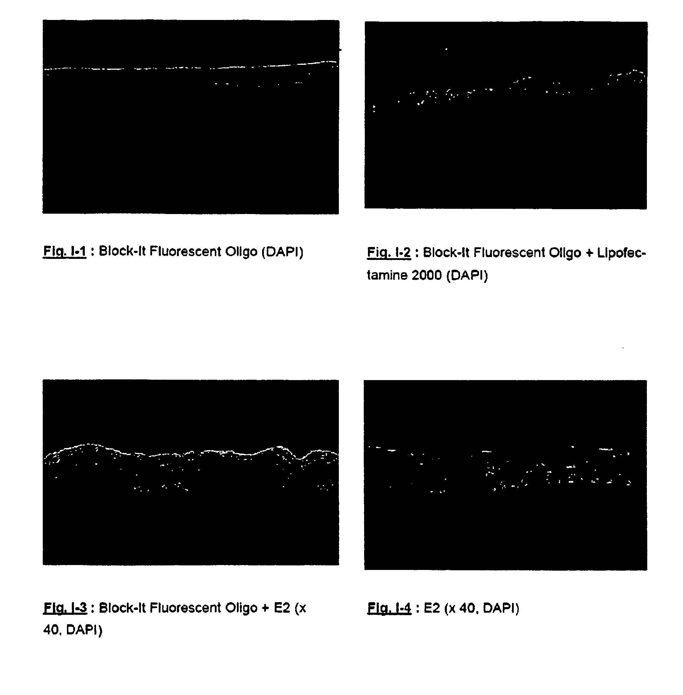

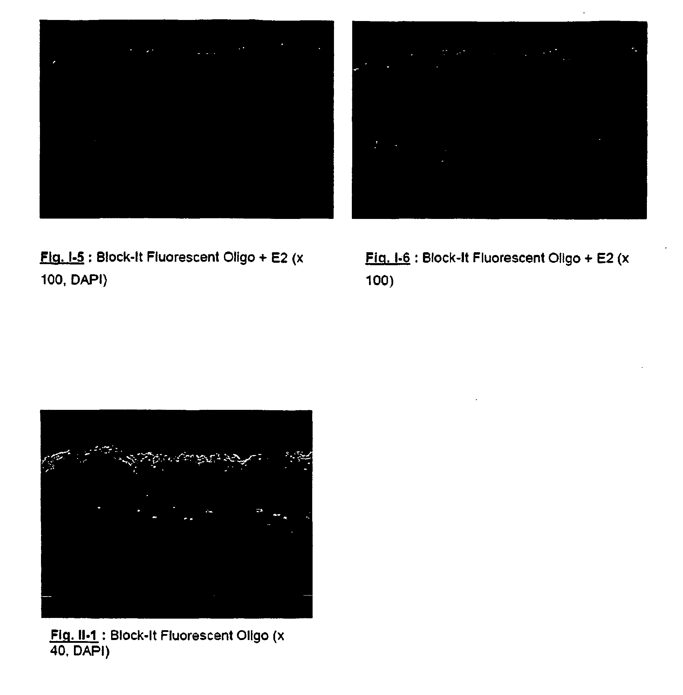

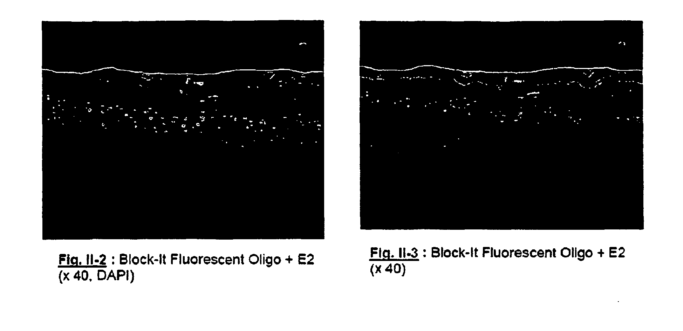

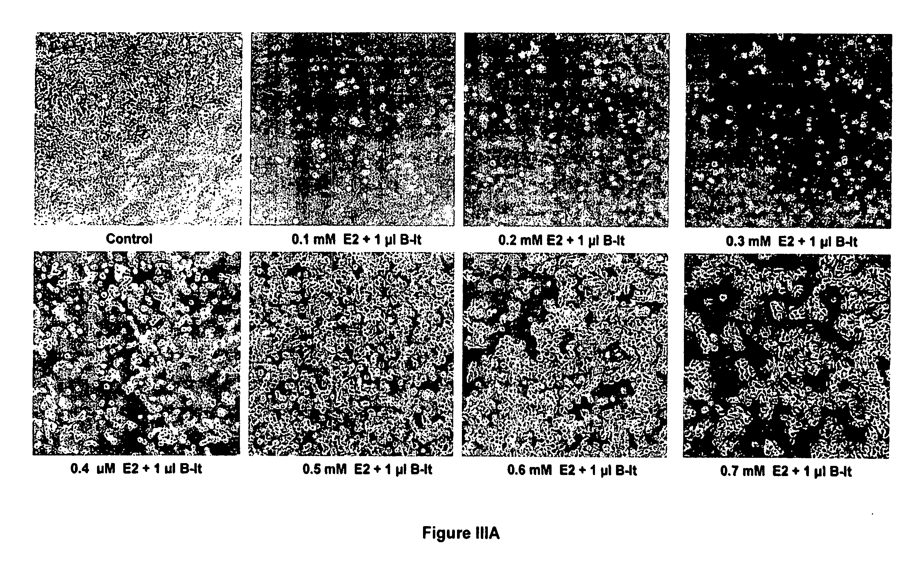

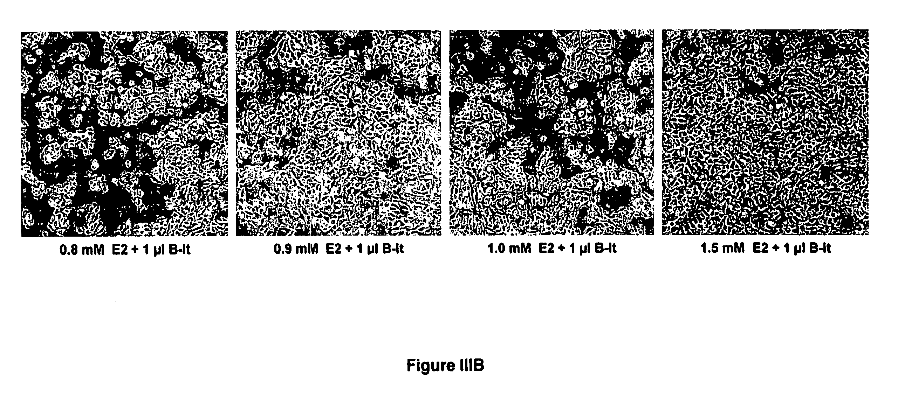

## Revendications

1. Composition topique ou oculaire comprenant au moins un oligonucléotide d'ARN double brin, **caractérisée en ce que** l'oligonucléotide d'ARN double brin adhère à la surface d'au moins une particule cationique d'une taille inférieure ou égale à 1 µm, de potentiel zeta compris entre 10 et 80 mV, ladite particule comprenant au moins un tensioactif cationique et étant choisie parmi les micelles de tensioactifs et les micelles de polymères blocs, ledit tensioactif cationique étant choisi parmi les sels d'ammonium quaternaire.

2. Composition selon la revendication 1, **caractérisée en ce que** la particule cationique a une taille inférieure ou égale à 500 nm.

3. Composition selon la revendication 1 ou 2, **caractérisée en ce que** la particule cationique a une taille inférieure ou égale à 300 nm.

4. Composition selon l'une quelconque des revendications précédentes, **caractérisée en ce que** la particule cationique est une micelle de tensioactifs amphiphiles non ioniques et de tensioactifs cationiques.

5. Composition selon l'une quelconque des revendications 1 à 3, **caractérisée en ce que** la micelle de polymère bloc est choisie parmi une micelle de polymère bloc amphiphile cationique, une micelle de polymère bloc amphiphile non ionique et de polymère bloc amphiphile cationique et une micelle de polymère bloc amphiphile non ionique et de tensioactif cationique.

6. Composition selon la revendication 1, **caractérisée en ce que** ledit sel d'ammonium quaternaire est choisi parmi :
- les sels d'ammonium quaternaire de formule générale (IV) suivante : 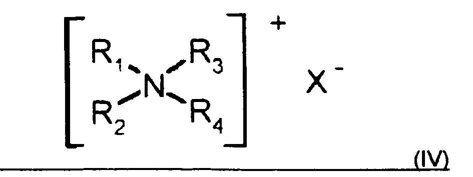 dans laquelle les radicaux R1 à R4, identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6), alkyl(C12-C22)amido alkyle(C2-C6), ou alkyl(C12-C22)acétate : et X est un anion choisi dans le groupe des halogénures, phosphates, acétates, lactates, alkyl(C2-C6)sulfates, alkyl-ou-alkylarylsulfonates,
- les sels d'ammonium quaternaire de l'imidazolinium,
- les sels de diammonium quaternaire de formule (VI) : 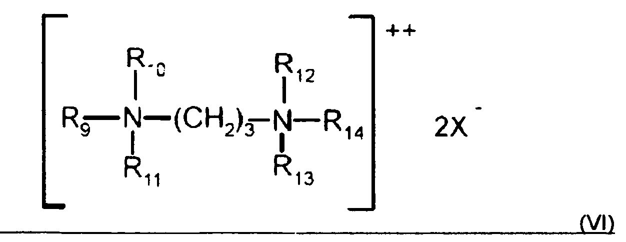 dans laquelle R9 désigne un radical aliphatique comportant environ de 16 à 30 atomes de carbone, R10, R11, R12, R13 et R14, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates,
- les sels d'ammonium quaternaire contenant au moins une fonction ester.

7. Composition selon la revendication 1, **caractérisée en ce que** ledit sel d'ammonium quaternaire est choisi parmi les sels d'ammonium quaternaire de formule générale (IV) suivante : 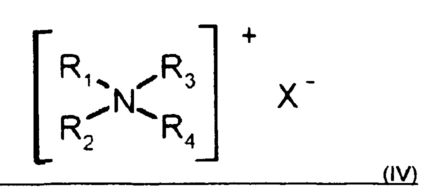 dans laquelle les radicaux R1 à R4, identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6), alkyl(C12-C22)amido alkyle(C2-C6), ou alkyl(C12-C22)acétate ; et X est un anion choisi dans le groupe des halogénures, phosphates, acétates, lactates, alkyl(C2-C6)sulfates, alkyl-ou-alkylarylsulfonates.

8. Utilisation topique ou oculaire d'une composition comprenant au moins un oligonucléotide d'ARN double brin, **caractérisé en ce que** l'oligonucléotide d'ARN double brin adhère à la surface d'au moins une particule cationique d'une taille inférieure ou égale à 1 µm, de potentiel zeta compris entre 10 et 80 mV, ladite particule comprenant au moins un tensioactif cationique et étant choisie parmi les micelles de tensioactifs et les micelles de polymères blocs, à des fins cosmétiques, ledit tensioactif cationique étant choisi parmi les sels d'ammonium quaternaire

9. Utilisation selon la revendication 8, **caractérisée en ce que** la particule cationique a une taille inférieure ou égale à 500 nm ou 300 nm.

10. Utilisation selon la revendication 8 **caractérisée en ce que** que ledit sel d'ammonium quaternaire est choisi parmi :
- les sels d'ammonium quaternaire de formule générale (IV) suivante : 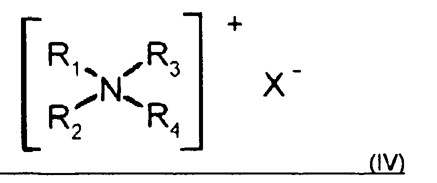 dans laquelle les radicaux R1 à R4 identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6) alkyl(C12-C22)amido alkyle(C2-C6), ou alkyl(C12-C22)acétate ; et X est un anion choisi dans le groupe des halogénures, phosphates, acétates, lactates, alkyl(C2-C6)sulfates, alkyl-ou-alkylarylsulfonates,
- les sels d'ammonium quaternaire de l'imidazolinium,
- les sels de diammonium quaternaire de formule (VI) : 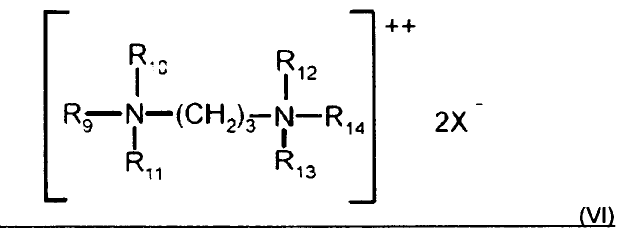 dans laquelle R9 désigne un radical aliphatique comportant environ de 16 à 30 atomes de carbone, R10, R11, R12, R13 et R14, identiques ou différents sont choisis parmi l'hydrogène
ou un radical alkyle comportant de 1 à 4 atomes de carbone, et X est un anion choisi dans le groupe des halogénures, acétates, phosphates, nitrates et méthylsulfates,
- les sels d'ammonium quaternaire contenant au moins une fonction ester.

11. Utilisation selon la revendication 8, **caractérisée en ce que** ledit sel d'ammonium quaternaire est choisi parmi les sels d'ammonium quaternaire de formule générale (IV) suivante : 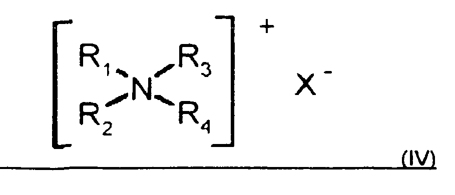 dans laquelle les radicaux R1 à R4, identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6), alkyl(C12-C22)amido alkyle(C2-C6), ou alkyl(C12-C22)acétate : et X est un anion choisi dans le groupe des halogénures, phosphates, acétates, lactates, alkyl(C2-C6)sulfates, alkyl-ou-alkylarylsulfonates.

12. Procédé de traitement cosmétique comprenant l'application topique d'une composition comprenant au moins un oligonucléotide d'ARN double brin, **caractérisé en ce que** l'oligonucléotide double brin adhère à la surface d'au moins une particule cationique d'une taille inférieure ou égale à 1 µm, de potentiel zeta compris entre 10 et 80 mV, ladite particule comprenant au moins un tensioactif cationique et étant choisie parmi les micelles de tensioactifs et les micelles de polymères blocs ledit tensioactif cationique étant choisi parmi les sels d'ammonium quaternaire.

13. Procédé selon la revendication 12, **caractérisé en ce que** l'application topique est précédée d'un prétraitement de la peau choisi parmi un stripping, un peeling chimique, une dermabrasion mécanique, l'application d'un ou plusieurs solvants ayant un effet délipidant, le nettoyage de la peau par un produit moussant détergent.

14. Procédé selon la revendication 12 ou 13, **caractérisé en ce que** l'application topique est suivie par un post traitement occlusif.

15. Procédé selon la revendication 12, **caractérisé en ce que** ledit sel d'ammonium quaternaire est choisiparmi :
- les sels d'ammonium quaternaire de formule générale (IV) suivante : 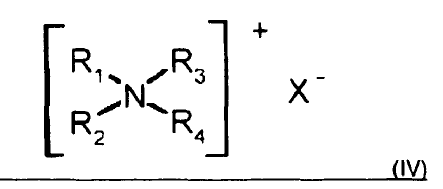 dans laquelle les radicaux R1 à R4, identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6), alkyl(C12-C22)amido alkyle(C2-C6), ou alkyl(C12-C22)acétate ; et X est un anion choisi dans le groupe des halogénures, phosphates, acétates, lactates, alkyl(C2-C6)sulfates, alkyl-ou-alkylarylsulfonates
- les sels d'ammonium Quaternaire de l'imidazolinium,
- les sels de diammonium quaternaire de formule (VI) : 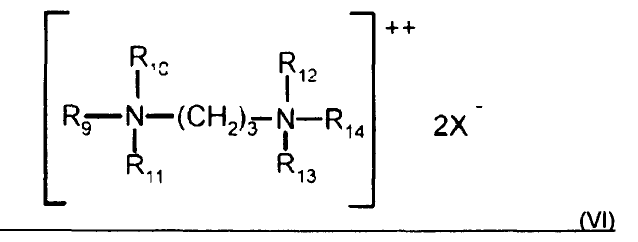 dans laquelle R9 désigne un radical aliphatique comportant environ de 16 à 30 atomes de carbone, R10, R11, R12, R13 et R14, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates,
- les sels d'ammonium quaternaire contenant au moins une fonction ester.

16. Procédé selon la revendication 12, **caractérisé en ce que** ledit sel d'ammonium quaternaire est choisi parmi les sels d'ammonium quaternaire de formule générale (IV) suivante : 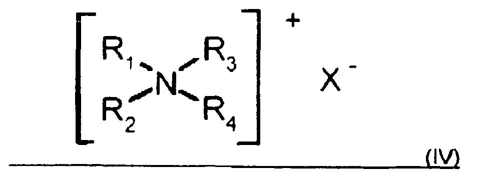 dans laquelle les radicaux R1 à R4, identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6), alkyl(C12-C22)amido alkyle(C2-C6), ou alkyl(C12-C22)acétate ; et X est un anion choisi dans le groupe des halogénures, phosphates, acétates, lactates, alkyl(C2-C6)sulfates, alkyl-ou-alkylarylsulfonates.

17. Utilisation de particules cationique d'une taille inférieure ou égale à 1 µm, de potentiel zeta compris entre 10 et 80 mV, ladite particule comprenant au moins un tensioactif cationique et étant choisie parmi les micelles de tensioactifs et, les micelles de polymères blocs, comme agent favorisant la pénétration topique ou oculaire d'au moins un oligonucléotide d'ARN double brin, ledit tensioactif cationique étant choisi parmi les sels d'ammonium quaternaire.

18. Utilisation selon la revendication 17, **caractérisée en ce que** ledit sel d'ammonium quaternaire est choisi parmi :
- les sels d'ammonium quaternaire de formule générale (IV) suivante : 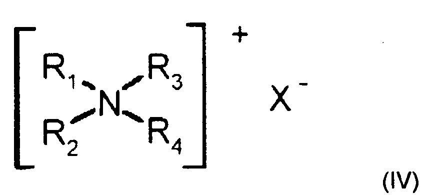 dans laquelle les radicaux R1 à R4, identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6), alkyl(C12-C22)amido alkyle(C2-C6), ou alkyl(C12-C22)acétate ; et X est un anion choisi dans le groupe des halogénures, phosphates, acétates, lactates, alkyl(C2-C6)sulfates, alkyl-ou-alkylarylsulfonates,
- les sels d'ammonium quaternaire de l'imidazolinium,
- les sels de diammonium quaternaire de formule (VI) : 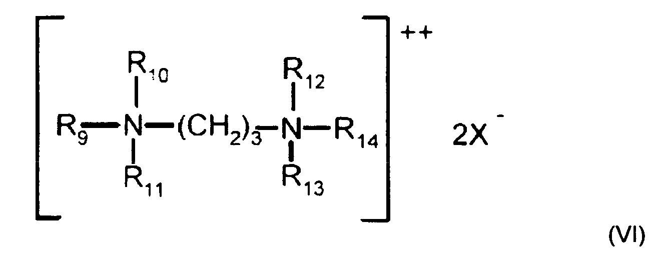 dans laquelle R9 désigne un radical aliphatique comportant environ de 16 à 30 atomes de carbone, R10, R11, R12, R13 et R14, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates,
les sels d'ammonium quaternaire contenant au moins une fonction ester.

19. Utilisation selon la revendication 17, **caractérisée en ce que** ledit sel d'ammonium quaternaire est choisi parmi les sels d'ammonium quaternaire de formule générale (IV) suivante : 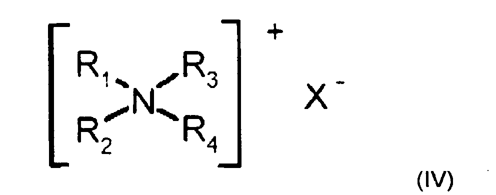 dans laquelle les radicaux R1 à R4, identiques ou différents, représentent un radical aliphatique, linéaire ou ramifié choisi parmi les radicaux alkyle, alcoxy, alkylamide ou hydroxyalkyle comportant de 1 à 30 atomes de carbone, les radicaux polyoxyalkylène(C2-C6), alkyl(C12-C22)amido alkyle(C2-C6), ou alkyl(C12-C22)acétate ; et X est un anion choisi dans le groupe des halogénures, phosphates, acétates, lactates, alkyl(C2-C6)sulfates, alkyl-ou-alkylarylsulfonates.

## Patentansprüche

1. Topische oder ophtalmische Zusammensetzung, die mindestens ein Doppelstrang-RNA-Oligonukleotid umfasst, **dadurch gekennzeichnet, dass** das Doppelstrang-RNA-Oligonukleotid an der Oberfläche von mindestens einem kationischen Partikel mit einer Größe von 1 µm oder darunter und mit einem zeta-Potential zwischen 10 und 80 mV haftet, wobei das Partikel mindestens ein kationisches Tensid umfasst und aus der Reihe der Tensidmizellen und der Blockpolymermizellen ausgewählt ist, wobei das kationische Tensid aus der Reihe der quartären Ammoniumsalze ausgewählt ist.

2. Zusammensetzung nach Anspruch 1, **dadurch gekennzeichnet, dass** das kationische Partikel eine Größe von 500 nm oder darunter aufweist.

3. Zusammensetzung nach Anspruch 1 oder 2, **dadurch gekennzeichnet, dass** das kationische Partikel eine Größe von 300 nm oder darunter aufweist.

4. Zusammensetzung nach einem der vorhergehenden Ansprüche, **dadurch gekennzeichnet, dass** es sich bei dem kationischen Partikel um eine Mizelle aus amphiphilen nichtionischen Tensiden und kationischen Tensiden handelt.

5. Zusammensetzung nach einem der Ansprüche 1 bis 3, **dadurch gekennzeichnet, dass** die Blockpolymermizelle aus der folgenden Reihe ausgewählt ist:
Mizelle aus einem kationischen amphiphilen Blockpolymer, Mizelle aus einem nichtionischen amphiphilen Blockpolymer und einem kationischen amphiphilen Blockpolymer und Mizelle aus einem nichtionischen amphiphilen Blockpolymer und einem kationischen Tensid.

6. Zusammensetzung nach Anspruch 1, **dadurch gekennzeichnet, dass** das Ammoniumsalz aus der folgenden Reihe ausgewählt ist:
- den quartären Ammoniumsalzen der folgenden allgemeinen Formel (IV): 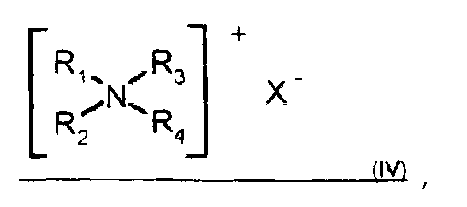 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet,
- den quartären Imidazoliniumammoniumsalzen,
- den quartären Diammoniumsalzen der Formel (VI): 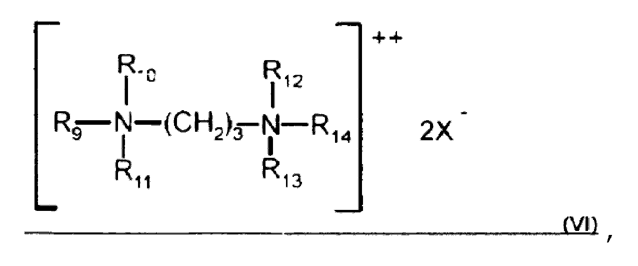 in der R9 einen aliphatischen Rest mit ungefähr 16 bis 30 Kohlenstoffatomen bedeutet, R10, R11, R12, R13 und R14, die gleich oder verschieden sind, aus der Reihe Wasserstoff oder Alkylrest mit 1 bis 4 Kohlenstoffatomen ausgewählt sind und X ein Anion, das aus der Gruppe der Halogenide, Acetate, Phosphate, Nitrate und Methylsulfate ausgewählt ist, bedeutet
- den quartären Ammoniumsalzen mit mindestens einer Esterfunktion.

7. Zusammensetzung nach Anspruch 1, **dadurch gekennzeichnet, dass** das quartäre Ammoniumsalz aus der Reihe der quartären Ammoniumsalze der folgenden allgemeinen Formel (IV) stammt: 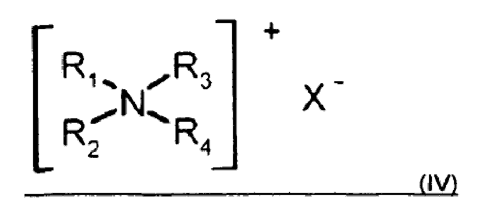 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet.

8. Topische oder ophtalmische Verwendung einer Zusammensetzung, die mindestens ein Doppelstrang-RNA-Oligonukleotid umfasst, **dadurch gekennzeichnet, dass** das Doppelstrang-RNA-Oligonukleotid an der Oberfläche von mindestens einem kationischen Partikel mit einer Größe von 1 µm oder darunter und mit einem zeta-Potential zwischen 10 und 80 mV haftet, wobei das Partikel mindestens ein kationisches Tensid umfasst und aus der Reihe der Tensidmizellen und der Blockpolymermizellen ausgewählt ist, für kosmetische Zwecke wobei das kationische Tensid aus der Reihe der quartären Ammoniumsalze ausgewählt ist.

9. Verwendung nach Anspruch 8, **dadurch gekennzeichnet, dass** das kationische Partikel eine Größe von 500 nm oder 300 nm oder darunter aufweist.

10. Verwendung nach Anspruch 8, **dadurch gekennzeichnet, dass** das quartäre Ammoniumsalz aus der folgenden Reihe ausgewählt ist:
- den quartären Ammoniumsalzen der folgenden allgemeinen Formel (IV): 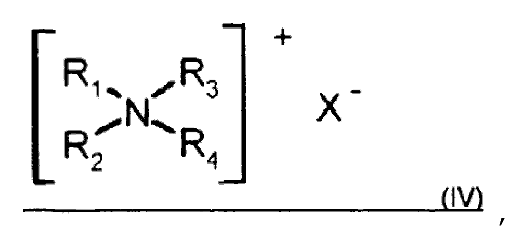 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet,
- den quartären Imidazoliniumammoniumsalzen,
- den quartären Diammoniumsalzen der Formel (VI): 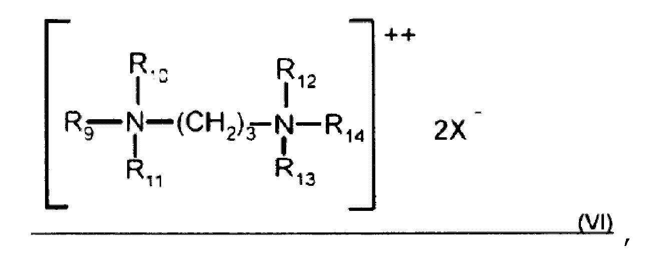 in der R9 einen aliphatischen Rest mit ungefähr 16 bis 30 Kohlenstoffatomen bedeutet, R10, R11, R12, R13 und R14, die gleich oder verschieden sind, aus der Reihe Wasserstoff oder Alkylrest mit 1 bis 4 Kohlenstoffatomen bedeuten und X ein Anion, das aus der Gruppe der Halogenide, Acetate, Phosphate, Nitrate und Methylsulfate ausgewählt ist, bedeutet
- den quartären Ammoniumsalzen mit mindestens einer Esterfunktion.

11. Verwendung nach Anspruch 8, **dadurch gekennzeichnet, dass** das quartäre Ammoniumsalz aus der Reihe der quartären Ammoniumsalze der folgenden allgemeinen Formel (IV) stammt: 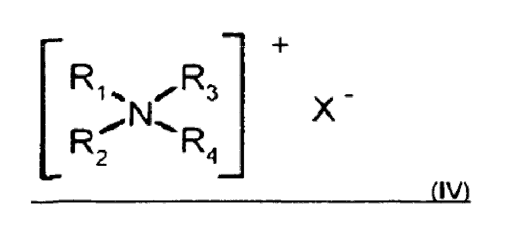 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet.

12. Kosmetisches Behandlungsverfahren, umfassend die topische Applikation einer Zusammensetzung, die mindestens ein Doppelstrang-RNA-Oligonukleotid umfasst, **dadurch gekennzeichnet, dass** das Doppelstrang-Oligonukleotid an der Oberfläche von mindestens einem kationischen Partikel mit einer Größe von 1 µm oder darunter und mit einem zeta-Potential zwischen 10 und 80 mV haftet, wobei das Partikel mindestens ein kationisches Tensid umfasst und aus der Reihe der Tensidmizellen und der Blockpolymermizellen ausgewählt ist, wobei das kationische Tensid aus der Reihe der quartären Ammoniumsalze ausgewählt ist.

13. Verfahren nach Anspruch 12, **dadurch gekennzeichnet, dass** vor der topischen Applikation eine Vorbehandlung der Haut, ausgewählt aus der Reihe Stripping, chemisches Peeling, mechanische Dermabrasion, Applikation von einem oder mehreren Lösungsmitteln mit entfettender Wirkung, Reinigung der Haut mit einem schäumenden Reinigungsprodukt erfolgt.

14. Verfahren nach Anspruch 12 oder 13, **dadurch gekennzeichnet, dass** auf die topische Applikation eine verschließende Behandlung erfolgt.

15. Verfahren nach Anspruch 12, **dadurch gekennzeichnet, dass** das quartäre Ammoniumsalz aus der folgenden Reihe ausgewählt ist:
- den quartären Ammoniumsalzen der folgenden allgemeinen Formel (IV): 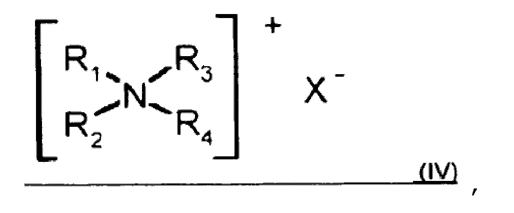 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet,
- den quartären Imidazoliniumammoniumsalzen,
- den quartären Diammoniumsalzen der Formel (VI): 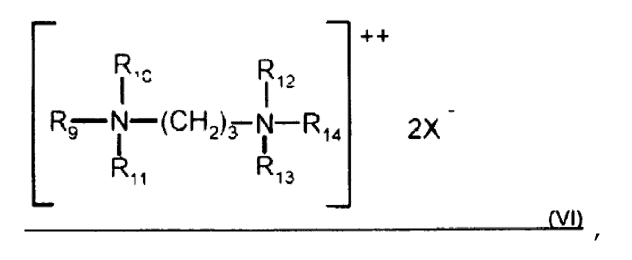 in der R9 einen aliphatischen Rest mit ungefähr 16 bis 30 Kohlenstoffatomen bedeutet, R10, R11, R12, R13 und R14, die gleich oder verschieden sind, aus der Reihe Wasserstoff oder Alkylrest mit 1 bis 4 Kohlenstoffatomen bedeuten und X ein Anion, das aus der Gruppe der Halogenide, Acetate, Phosphate, Nitrate und Methylsulfate ausgewählt ist, bedeutet
- den quartären Ammoniumsalzen mit mindestens einer Esterfunktion.

16. Verfahren nach Anspruch 12, **dadurch gekennzeichnet, dass** das quartäre Ammoniumsalz aus der Reihe der quartären Ammoniumsalze der folgenden allgemeinen Formel (IV) stammt: 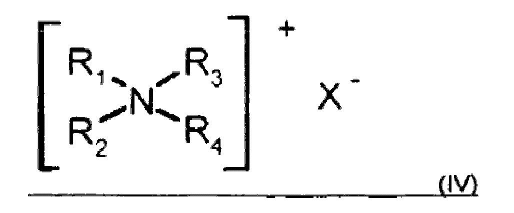 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet.

17. Verwendung von kationischen Partikeln mit einer Größe von 1 µm oder darunter und einem zeta-Potential von zwischen 10 und 80 mV, wobei das Partikel mindestens ein kationisches Tensid umfasst, das aus der Reihe Tensidmizellen und Blockpolymermizellen stammt, als Mittel zum Begünstigen der topischen oder ophtalmischen Eindringung von mindestens einem Doppelstrang-RNA-Oligonukleotid, wobei das kationische Tensid aus der Reihe der quartären Ammoniumsalze stammt.

18. Verwendung nach Anspruch 17, **dadurch gekennzeichnet, dass** das quartäre Ammoniumsalz aus der folgenden Reihe ausgewählt ist:
- den quartären Ammoniumsalzen der folgenden allgemeinen Formel (IV): 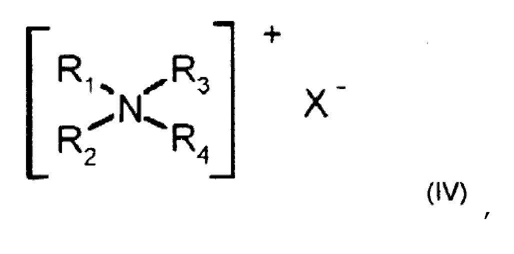 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet,
- den quartären Imidazoliniumammoniumsalzen,
- den quartären Diammoniumsalzen der Formel (VI): 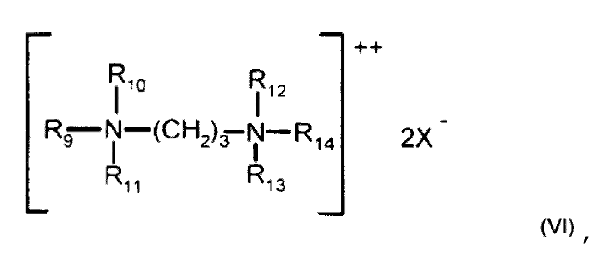 in der R9 einen aliphatischen Rest mit ungefähr 16 bis 30 Kohlenstoffatomen bedeutet, R10, R11, R12, R13 und R14, die gleich oder verschieden sind, aus der Reihe Wasserstoff oder Alkylrest mit 1 bis 4 Kohlenstoffatomen bedeuten und X ein Anion, das aus der Gruppe der Halogenide, Acetate, Phosphate, Nitrate und Methylsulfate ausgewählt ist, bedeutet,
- den quartären Ammoniumsalzen mit mindestens einer Esterfunktion.

19. Verwendung nach Anspruch 17, **dadurch gekennzeichnet, dass** das quartäre Ammoniumsalz aus der Reihe der quartären Ammoniumsalze der folgenden allgemeinen Formel (IV) stammt: 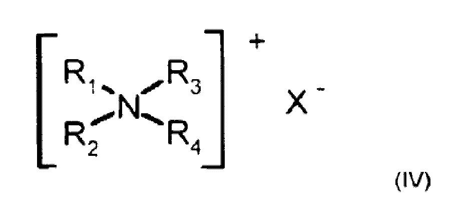 in der die Reste R1 bis R4, die gleich oder verschieden sind, einen geradkettigen oder verzweigten aliphatischen Rest, ausgewählt aus den Alkyl-, Alkoxy-, Alkylamid- oder Hydroxyalkylresten mit 1 bis 30 Kohlenstoffatomen, den (C2-C6)Polyoxyalkylenresten, den (C2-C6)Alkylamido(C12-C22)alkylresten oder (C12-C22)Alkylacetatresten bedeuten; und X ein Anion, das aus der Gruppe der Halogenide, Phosphate, Acetate, Lactate, (C2-C6)Alkylsulfate, Alkyl- oder Alkylarylsulfonate ausgewählt ist, bedeutet.

## Claims

1. Topical or ocular composition comprising at least one double-stranded RNA oligonucleotide, **characterized in that** the double-stranded RNA oligonucleotide adheres to the surface of at least one cationic particle less than or equal to 1 µm in size, with a zeta potential of between 10 and 80 mV, said particle comprising at least one cationic surfactant and being chosen from surfactant micelles and block polymer micelles, said cationic surfactant being chosen from quaternary ammonium salts.

2. Composition according to Claim 1, **characterized in that** the cationic particle is less than or equal to 500 nm in size.

3. Composition according to Claim 1 or 2, **characterized in that** the cationic particle is less than or equal to 300 nm in size.

4. Composition according to any one of the preceding claims, **characterized in that** the cationic particle is a micelle of non-ionic amphiphilic surfactants and of cationic surfactants.

5. Composition according to any one of Claims 1 to 3, **characterized in that** the block polymer micelle is chosen from a micelle of cationic amphiphilic block polymer, a micelle of non-ionic amphiphilic block polymer and of cationic amphiphilic block polymer and a micelle of non-ionic amphiphilic block polymer and of cationic surfactant.

6. Composition according to Claim 1, **characterized in that** said quaternary ammonium salt is chosen from:
- the quaternary ammonium salts of general formula (IV) below: 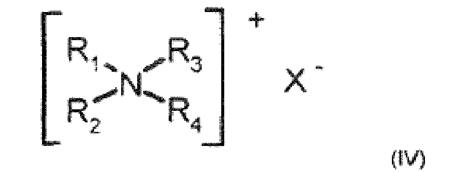 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂) alkylamido (C₂-C₆)alkyl radicals or (C₁₂-C₂₂) alkyl acetate radicals; and X is an anion chosen from the group of halides, phosphates, acetates, lactates, (C₂-C₆)alkyl sulfates, alkyl sulfonates or alkylaryl sulfonates,
- quaternary ammonium salts of imidazolinium,
- the diquaternary ammonium salts of formula (VI): 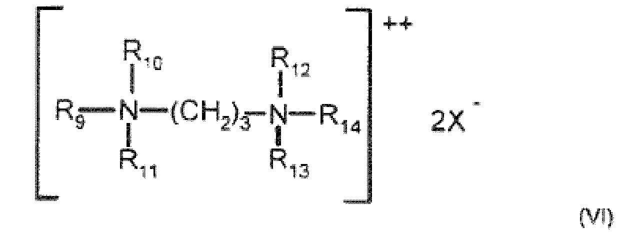 in which R9 denotes an aliphatic radical containing approximately from 16 to 30 carbon atoms, R10, R11, R12, R13 and R14, which may be identical or different, are chosen from hydrogen or an alkyl radical containing from 1 to 4 carbon atoms, and X is an anion chosen from the group of halides, acetates, phosphates, nitrates and methyl sulfates,
- quaternary ammonium salts containing at least one ester function.

7. Composition according to Claim 1, **characterized in that** said quaternary ammonium salt is chosen from the quaternary ammonium salts of general formula (IV) below: 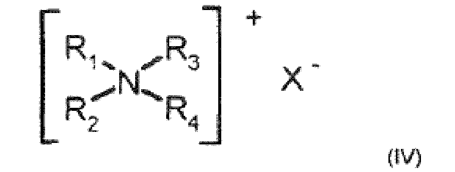 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂)alkylamido(C₂-C₆)alkyl radicals or (C₁₂-C₂₂) alkyl acetate radicals; and X is an anion chosen from the group of halides, phosphates, acetates, lactates, (C₂-C₆) alkyl sulfates, alkyl sulfonates or alkylaryl sulfonates.

8. Topical or ocular use of a composition comprising at least one double-stranded RNA oligonucleotide, **characterized in that** the double-stranded RNA oligonucleotide adheres to the surface of at least one cationic particle less than or equal to 1 µm in size, with a zeta potential of between 10 and 80 mV, said particle comprising at least one cationic surfactant and being chosen from surfactant micelles and block polymer micelles, for cosmetic purposes, said cationic surfactant being chosen from quaternary ammonium salts.

9. Use according to Claim 8, **characterized in that** the cationic particle is less than or equal to 500 nm or 300 nm in size.

10. Use according to Claim 8, **characterized in that** said quaternary ammonium salt is chosen from:
- the quaternary ammonium salts of general formula (IV) below: 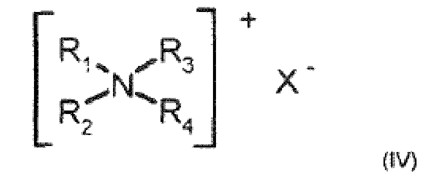 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂) alkylamido (C₂-C₆)alkyl radicals or (C₁₂-C₂₂) alkyl acetate radicals; and X is an anion chosen from the group of halides, phosphates, acetates, lactates, (C₂-C₆)alkyl sulfates, alkyl sulfonates or alkylaryl sulfonates,
- quaternary ammonium salts of imidazolinium,
- the diquaternary ammonium salts of formula (VI): 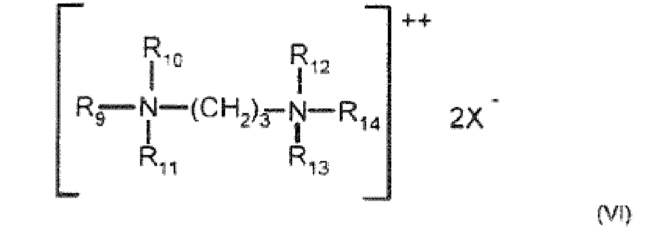 in which R9 denotes an aliphatic radical containing approximately from 16 to 30 carbon atoms, R10, R11, R12, R13 and R14, which may be identical or different, are chosen from hydrogen or an alkyl radical containing from 1 to 4 carbon atoms, and X is an anion chosen from the group of halides, acetates, phosphates, nitrates and methyl sulfates,
- quaternary ammonium salts containing at least one ester function.

11. Use according to Claim 8, **characterized in that** said quaternary ammonium salt is chosen from the quaternary ammonium salts of general formula (IV) below: 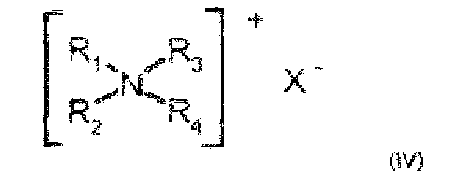 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂)alkylamido(C₂-C₆)alkyl radicals or (C₁₂-C₂₂) alkyl acetate radicals; and X is an anion chosen from the group of halides, phosphates, acetates, lactates, (C₂-C₆) alkyl sulfates, alkyl sulfonates or alkylaryl sulfonates.

12. Cosmetic treatment process comprising the topical application of a composition comprising at least one double-stranded RNA oligonucleotide, **characterized in that** the double-stranded oligonucleotide adheres to the surface of at least one cationic particle less than or equal to 1 µm in size, with a zeta potential of between 10 and 80 mV, said particle comprising at least one cationic surfactant and being chosen from surfactant micelles and block polymer micelles, said cationic surfactant being chosen from quaternary ammonium salts.

13. Process according to Claim 12, **characterized in that** the topical application is preceded by a pretreatment of the skin chosen from stripping, chemical peeling, mechanical dermabrasion, the application of one or more solvents having a defatting effect, and cleansing of the skin with a detergent foaming product.

14. Process according to Claim 12 or 13, **characterized in that** the topical application is followed by an occlusive post-treatment.

15. Process according to Claim 12, **characterized in that** said quaternary ammonium salt is chosen from:
- the quaternary ammonium salts of general formula (IV) below: 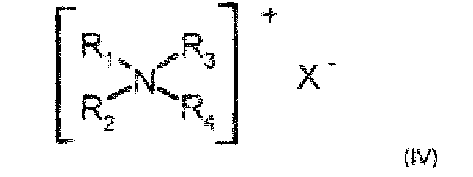 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂) alkylamido (C₂-C₆)alkyl radicals or (C₁₂-C₂₂) alkyl acetate radicals; and X is an anion chosen from the group of halides, phosphates, acetates, lactates, (C₂-C₆)alkyl sulfates, alkyl sulfonates or alkylaryl sulfonates,
- quaternary ammonium salts of imidazolinium,
- the diquaternary ammonium salts of formula (VI): 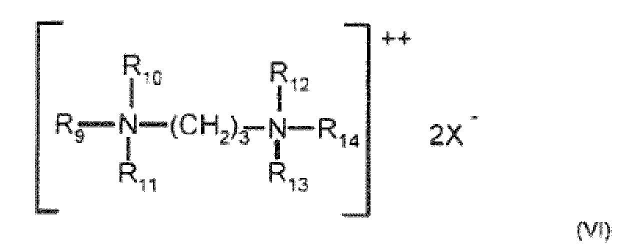 in which R9 denotes an aliphatic radical containing approximately from 16 to 30 carbon atoms, R10, R11, R12, R13 and R14, which may be identical or different, are chosen from hydrogen or an alkyl radical containing from 1 to 4 carbon atoms, and X is an anion chosen from the group of halides, acetates, phosphates, nitrates and methyl sulfates,
- quaternary ammonium salts containing at least one ester function.

16. Process according to Claim 12, **characterized in that** said quaternary ammonium salt is chosen from the quaternary ammonium salts of general formula (IV) below: 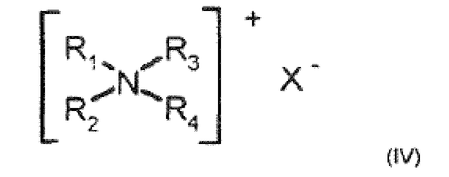 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂)alkylamido(C₂-C₆)alkyl radicals or (C₁₂-C₂₂) alkyl acetate radicals; and X is an anion chosen from the group of halides, phosphates, acetates, lactates, (C₂-C₆)alkyl sulfates, alkyl sulfonates or alkylaryl sulfonates.

17. Use of cationic particles less than or equal to 1 µm in size, with a zeta potential of between 10 and 80 mV, said particle comprising at least one cationic surfactant and being chosen from surfactant micelles and block polymer micelles, as an agent which promotes the topical or ocular penetration of at least one double-stranded RNA oligonucleotide, said cationic surfactant being chosen from quaternary ammonium salts.

18. Use according to Claim 17, **characterized in that** said quaternary ammonium salt is chosen from:
- the quaternary ammonium salts of general formula (IV) below: 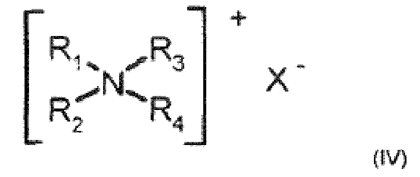 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂) alkylamido (C₂-C₆)alkyl radicals or (C₁₂-C₂₂) alkyl acetate radicals; and X is an anion chosen from the group of halides, phosphates, acetates, lactates, (C₂-C₆)alkyl sulfates, alkyl sulfonates or alkylaryl sulfonates,
- quaternary ammonium salts of imidazolinium,
- the diquaternary ammonium salts of formula (VI): 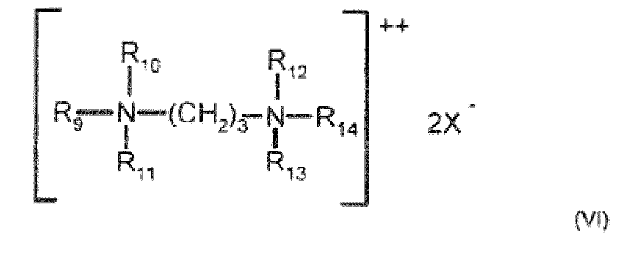 in which R9 denotes an aliphatic radical containing approximately from 16 to 30 carbon atoms, R10, R11, R12, R13 and R14, which may be identical or different, are chosen from hydrogen or an alkyl radical containing from 1 to 4 carbon atoms, and X is an anion chosen from the group of halides, acetates, phosphates, nitrates and methyl sulfates,
- quaternary ammonium salts containing at least one ester function.

19. Use according to Claim 17, **characterized in that** said quaternary ammonium salt is chosen from the quaternary ammonium salts of general formula (IV) below: 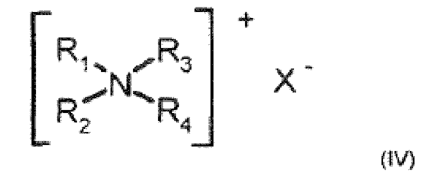 in which the radicals R1 to R4, which may be identical or different, represent a linear or branched aliphatic radical chosen from alkyl, alkoxy, alkylamide or hydroxyalkyl radicals containing from 1 to 30 carbon atoms, polyoxy(C₂-C₆)alkylene radicals, (C₁₂-C₂₂)alkylamido(C₂-C₆)alkyl radicals or (C₁₂-C₂₂) alkyl acetate radicals; and X is an anion chosen from the group of halides, phosphates, acetates, lactates, (C₂-C₆) alkyl sulfates, alkyl sulfonates or alkylaryl sulfonates.
